(19) Europäisches Patentamt
European Patent Office
Office européen des brevets

(11) Publication number: **0 008 145**
**B1**

(12) **EUROPEAN PATENT SPECIFICATION**

(45) Date of publication of patent specification: **01.02.84**

(21) Application number: **79200424.4**

(22) Date of filing: **30.07.79**

(51) Int. Cl.³: **C 07 D 213/82,**
C 07 D 213/83,
C 07 D 213/78,
C 07 D 213/57,
C 07 D 213/81,
C 07 D 213/53,
C 07 D 401/06,
A 01 N 43/40

(54) **Use of phenyliminomethylpyridine derivatives as fungicides and plant growth regulants compositions containing such derivatives and phenyliminomethylpyridine derivatives and their preparation.**

(30) Priority: **08.08.78 GB 3252378**

(43) Date of publication of application:
**20.02.80 Bulletin 80/4**

(45) Publication of the grant of the patent:
**01.02.84 Bulletin 84/5**

(84) Designated Contracting States:
**AT BE CH DE FR GB IT LU NL**

(56) References cited:
**DE - A - 2 611 601**
**FR - A - 1 171 911**
**FR - A - 2 168 931**
**FR - M - 6 295**
**US - A - 3 697 505**

**JOURNAL OF HETEROCYCLIC CHEMISTRY, vol. 12, 1975, pages 143-146 State St. Orem, Utah, USA PANKAJA K. KADABA: "Triazolines VIII. Action of Diazoalkanes on Heterocyclic Substituted Schiff Bases".**

**AUSTRALIAN JOURNAL OF CHEMISTRY, vol. 26, 1973, pages 1031-1041. Melbourne, AU. C.S. BARNES et al.: "Mass spectra of nitrogen heterocycles. Pyridyl schiff bases"**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Ten Haken, Pieter**
**"Konkelboet", The Street**
**Eastling, Near Faversham Kent (GB)**
Inventor: **Webb, Shirley Beatrice**
**17 North Street, Ashford Road**
**Sheldwich, Near Faversham Kent (GB)**
Inventor: **Smith, Graham Clifford**
**3 Beaconsfield Road**
**Sittingbourne, Kent (GB)**

(74) Representative: **Hunter, Keith Roger Ian et al,**
**4 York Road**
**London SE1 7NA (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 008 145 B1**

Use of phenyliminomethylpyridine derivatives as fungicides and plant growth regulants, compositions containing such derivatives, phenyliminomethylpyridine derivatives and their preparation

The present invention relates to certain phenyliminomethylpyridine derivatives. It has been found that many of these derivatives have pesticidal and plant growth regulating properties. Some of them exhibit a notable fruit abscission activity. Of particular interest are those compounds possessing fungicidal and herbicidal activity.

The invention therefore relates to the use as a fungicide or plant growth regulant of such derivatives and to pesticidal compositions comprising one or more of such derivatives.

The invention also relates to novel phenyliminomethylpyridine derivatives, and their preparation.

Accordingly the present invention provides the use as fungicide and/or a plant growth regulant of a compound of formula:

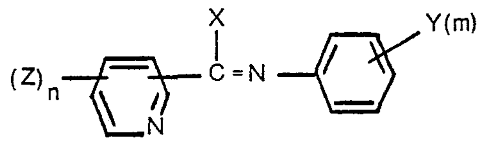

(I)

wherein X represents a hydrogen atom, a cyano group, a non-substituted or a halo- or alkoxy-substituted alkoxy-group, a cycloalkloxy group, an alkylthio, carboxymethylthio, phenylthio, 2-chlorophenylthio, 4-chlorophenylthio, 4-bromophenylthio, 2,4-dichlorophenylthio, an alkyl group or a group nrr′, in which R is a hydrogen atom or an alkyl group and R′ is an alkyl, a phenyl or a cycloalkyl group and when R is hydrogen can R′ be p-fluorophenyl, benzyl, diisopropylamino ethyl, methoxy ethyl or hydroxy ethyl or R and R′ together with the interjacent nitrogen atom may form a heterocyclic ring chosen from triazole, pyrolidine, morpholine, piperidine, or 3,5-dimethylpiperidine, Y and Z independently are halogen atom, alkyl-, alkoxy- or nitro groups and m and n are each 0, 1 or 2, whereby the same or different groups Y may be present when m equals 2 and the same or different groups Z when n equals 2.

Among the compounds of the above formula the 3-pyridyl derivatives are preferred in view of their fungicidal properties. The pyridyl group may be non-substituted or substituted, suitably with one or more halogen atoms, such as fluorine, chlorine or bromine atoms, alkyl groups, such as methyl- or ethyl groups, alkoxy groups, such as methoxy-, ethoxy- or isopropoxy groups or nitro groups. Halogen atoms, in particular chlorine atoms are preferred substituents.

Preferred compounds of the above formula are those in which X represents an alkoxy group or a substituted alkoxy group. Examples of suitable alkoxy groups are methoxy-, ethoxy-, propoxy-, isopropoxy-, n-butyoxy-, 2-butoxy, isobutoxy-, ter.butoxy-, octyloxy-, decyloxy-, dodecyloxy- and eicosyloxy groups. Suitable substituted alkoxy groups include those substitued with halogen atoms, in particular chlorine atoms, acyloxy groups and alkoxy groups, such as methoxy- and ethoxy groups. A notable fungicidal activity has further been observed with compounds in which X represents a group —NRR′. In particular preferred are compounds wherein X is a dialkylamino group, such as a dibutylamino-, dipropylamine- or ethylpropylamino group or a mono-alkylamino group having at least 4 and preferably at least 6 carbon atoms in the alkyl group, such as a n-butylamino- or n-nonylamino group. Suitable —NRR′ groups wherein R and R′ together with the nitrogen atom form a heterocyclic ring are, for example, piperidinyl- and triazoyl groups.

In compounds wherein X represents an alkylthio group, the alkyl moiety usually contains from 1—8, preferably from 1—5 carbon atoms, suitable alkyl moieties including optionally substituted methyl-, isopropyl- and tert. butyl groups. Examples of suitable arylthio groups are a phenylthio-, 2-chlorophenylthio-, 4-chlorophenylthio- or a 4-bromophenylthio group and dihaloarylthio groups, e.g. a 2,4-dichlorophenylthio group.

The phenyl group in the compounds of formula I above may be non-substituted or may be substituted with one or two groups Y. In the event m equals 2, the groups Y may be different if so desired; usually, however, the compounds have two identical substituents in the phenyl group; as a rule the latter compounds are more readily prepared.

Preferably Y represents a halogen atom, in particular a chlorine atom.

Suitable substituents of the phenyl group in the compounds of the above formula are those recommended as substituents of the pyridyl group.

It has been found that the following compounds are very suitable to be applied for use as fungicides

4′-chlorophenylimino-C-(cyano)methyl-3-pyridine;
4′-chlorophenylimino-C-(n-propoxy)methyl-3-pyridine;
4′-chlorophenylimino-C-(n-butoxy)methyl-3-pyridine;
4′-chlorophenylimino-C-(cyclohexyloxy)methyl-3-pyridine;
4′-chlorophenylimino-C-(methylthio)methyl-3-pyridine;
4′-chlorophenylimino-C-(tert.butylthio)methyl-3-pyridine;
4′-chlorophenylimino-C-(di-n-butylamino)methyl-3-pyridine;

4'chlorophenylimino-C-(N-piperidinyl)methyl-3-pyridine.

For use as fungicides or plant growth regulators the compounds may be employed alone or in the form of a composition. Accordingly the invention provides a pesticidal and/or plant growth regulating composition comprising a surface active agent and optionally a carrier, or a solid carrier and as pesticidal and/or plant growth regulating ingredient at least one compound of formula I as defined above, provided that when X is hydrogen, n is 0 or Z is methyl and n is 1 and the derivative is a 2- or 4-pyridyl derivative then Y is not methyl, methoxy, ethoxy, or chloro; and when X is hydrogen and the derivative is a 6-methyl-2-pyridyl (n = 1) derivative or a 4-pyridyl (n = 0) derivative then m is not 0.

The compounds of formula I as described above are, so far as is known, with the exception of a small group or compounds, all novel. Accordingly the present invention further provides phenyliminomethylpyridine derivatives of formula I in claim 1 as defined in claim 14, provided that when X is hydrogen, and n = 0, then m is not 0, or, when Y is chloro or methoxy, is not 1; and when X is hydrogen, the derivative is a 3- or 4-pyridyl derivative, and m = 1, then Y is not a 3-nitro substituent and when X is a cyano group, the derivative is a 4-pyridyl derivative, and n = 0 and m = 1, then Y is other than a 4-methoxy substituent.

The compounds according to the invention may be prepared by any suitable adaptation of known methods. A preferred method for the preparation of compounds of formula I other than those in which X is hydrogen or alkyl, containing an optionally substituted 3-pyridyl group consists in reacting an, optionally substituted, nicotinic anilide with a thionyl halide, preferably thionyl chloride and converting the formed phenylimino-C-(halo)methyl-3-pyridine to the desired product by reaction with a compound QX, wherein Q is hydrogen, an alkali metal or copper and X represents one of the afore-mentioned group, with the exception of hydrogen and alkyl.

The term 'carrier' as used herein means a material, which may be inorganic or organic and of synthetic or natural origin, with which the active compound is mixed or formulated to facilitate its application to the plant, seed, soil or other object to be treated, or its storage, transport or handling. The carriers may be solid or fluid. At least one of the carriers may be a surface-active agent. Any of the material usually applied in formulating fungicides may be used as carrier.

Suitable solid carriers are natural and synthetic clays and silicates, for example natural silicas, such as diatomaceous earth; magnesium silicates, for example, talcs, magnesium aluminium silicates, for example, attapulgites and vermiculiates; aluminium silicates, for example, kaolinites, montmorillonites and micas; calcium carbonates; calcium sulphate; synthetic hydrated silicon oxides and synthetic calcium or aluminium silicates; elements, such as for example, carbon and sulphur; natural and synthetic resins, such as for example, coumarone resins, polyvinyl chloride and styrene polymers and copolymers; solid polychlorophenyls; bitumen; waxes, such as for example, beeswax, paraffin wax, and chlorinated mineral waxes; and solid fertilizers, for example superphosphates.

Examples of suitable fluid carriers are water, alcohols, such as for example, isopropanol, glycols; ketones, such as for example, acetone, methyl ethyl ketone, methyl isobutyl ketone and cyclohexanone; ethers; aromatic hydrocarbons, such as for example, benzene, toluene and xylene; petroleum fractions, such as for example, kerosine, light mineral oils; chlorinated hydrocarbons, such as for example, carbon tetrachloride, perchloroethylene, trichloroethane, including liquefied normally vaporous gaseous compounds. Mixtures of different liquids are often suitable.

The surface-active agent may be an emulsifying agent or a dispersing agent or a wetting agent, it may be non-ionic or ionic. Any of the surface-active agents usually applied in formulating fungicides may be used. Examples of suitable surface-active agents are the sodium or calcium salts of polyacrylic acids and lignin sulphonic acids; the condensation products of fatty acids or aliphatic amines or amides containing at least 12 carbon atoms in the molecule with ethylene oxide and/or propylene oxide; fatty acid esters or glycerol, sorbitan, sucrose or pentaerythritol; condensates of these with ethylene oxide and/or propylene oxide; condensation products of fatty alcohols or alkyl phenols, for example p-octylphenol or p-octylcresol, with ethylene oxide and/or propylene oxide; sulphates or sulphonates of these condensation products; alkali or alkaline earth metal salts, preferably sodium salts, of sulphuric or sulphonic acid esters containing at least 10 carbon atoms in the molecule, for example, sodium lauryl sulphate, sodium secondary alkyl sulphates, sodium salts or sulphonated castor oil, and sodium alkylaryl sulphonates, such as sodium dodecyl-benzene sulphonate; and polymers of ethylene oxide and copolymers of ethylene oxide and propylene oxide.

The compositions of the invention may be formulated as wettable powders, dusts, granules, solutions, emulsifiable concentrates, emulsions, suspension concentrates and aerosols and will generally contain 0.5 to 95%w, preferably 0.5 to 75%w, of toxicant. Wettable powders are usually compounded to contain 25, 50 or 75%w of toxicant and usually contain, in addition to solid carrier, 3—10%w of a dispersing agent and, where necessary, 0—10%w of stabilizer(s) and/or other additives, such as penetrants or stickers. Dusts are usually formulated as a dust concentrate having a similar composition to that of a wettable powder but without a dispersant and are diluted in the field with further solid carrier to give a composition usually containing $\frac{1}{2}$—10%w of toxicant. Granules are usually prepared to have a size between 10 and 100 BS mesh (1.676—0.152 mm), and may be manufactured by agglomeration or impregnation techniques. Generally, granules will contain $\frac{1}{2}$—25%w toxicant and 0—10%w of additives, such as stabilizers, slow-release modifiers and binding agents. Emulsifiable

concentrates usually contain, in addition to the solvent and, when necessary, co-solvent, 10—50%w/v toxicant, 2—20%w/v emulsifiers and 0—20%w/v of appropriate additives, such as stabilizers, penetrants and corrosion inhibitors. Suspension concentrates are compounded so as to obtain a stable, non-sedimenting, flowable product and usually contain 10—75%w toxicant, 0.5—15%w of dispersing agent, 0.1—10%w of suspendingg agents, such as protective colloids and thixotropic agents, 0—10%w of appropriate additives, such as defoamers, corrosion inhibitors, stabilizers, penetrants and stickers, and as carrier, water or an organic liquid in which the toxicant is substantially insoluble, certain organic solids or inorganic salts may be dissolved in the carrier to assist in preventing sedimentation or as anti-freeze agents for water.

The compositions of the invention may contain other ingredients, for example protective colloids, such as gelatin, glue, casein, gums, cellulose ethers, and polyvinyl alcohol; thixotropic agents, e.g., bentonites, sodium polyphosphates; stabilizers, such as ethylene diamine tetra-acetic acid, urea, triphenyl phosphate; other fungicides or pesticides; and stickers, for example non-volatile oils.

Aqueous dispersion and emulsions, for example, compositions obtained by diluting a wettable powder or an emulsifiable concentrate according to the invention with water, also lie within the scope of the present invention. The said emulsions may be of the water-in-oil or fo the oil-in-water type, and may have a thick "mayonaise"-like consistency.

The invention is illustrated in the following Examples:

Example 1

*Preparation of 4'-chlorophenylimino-C-(cyano)methyl-3-pyridine*

A stirred mixture of nicotinic-4'-chloroanilide (4.65 g, 0.02 mol) and thionyl chloride (20 ml) was heated under reflux for 2 hours. Excess thionyl chloride was removed *in vacuo*, and the residue treated with dry pyridine (50 ml). To the stirred solution was added dry cuprous cyanide (3.58 g, 0.04 mole). After 15 min., pyridine was removed *in vacuo*, and the residue was extracted with warm acetone. Evaporation of the solvent from the extract gave the 4'-chlorophenylimino-C-(cyano)methyl-3-pyridine in 60% yield as a yellow crystalline solid, melting point 73—75°C.

*Analysis:*

| | |
|---|---|
| Calculated for $C_{13}H_8N_3Cl$ | : C 64.6; H 3.31; N 17.39% |
| Found | : C 63.7; H 3.3; N 17.0% |

Example 2

*Preparation of 4'-chlorophenylimino-C-(isopropxy)methyl-3-pyridine*

A stirred mixture of nicotinic-4'-chloroanilide (7 g, 0.03 mole) and thionyl chloride (22.5 ml) was heated under reflux for 2 hours. Excess thionyl chloride was removed *in vacuo*, and the residue suspended in dry dimethoxyethane (75 ml). A solution of sodium (2.43 g, 0.105 mole) in dry isopropanol (100 ml) was added all at once, and the mixture was stirred for 1h at room temperature and then heated under reflux for 16 hours. Solvents were removed *in vacuo*, and the residue treated with ether; the ethereal solution was washed with water (2 ×) and dried over $MgSO_4$. After removal of the solvent the residue was subjected to column chromatography on silica gel, eluting the $Et_2O$/hexane (1:1). The 4'-chlorophenylimino-C-(isopropoxy)methyl-3-pyridine was obtained, after recrystallization from 60/80 petroleum spirit, in 27.5% yield as a colourless crystalline solid, melting point 71—72.5°C.

*Analysis:*

| | |
|---|---|
| Calculated for $C_{15}H_{15}N_2OCl$: | C 65.6; H 5.5; N 10.2% |
| Found | : C 65.4; H 5.8; N 10.0% |

Example 3

*Preparation of 4'-chlorophenylimino-C-(*n*-propoxy)methyl-3-pyridine*

A stirred mixture of nicotinic-4'-chloroanilide (7 g, 0.03 mole) and thionyl chloride (22.5 ml) was heated under reflux for 2 hours. Excess thionyl chloride was removed *in vacuo*, and the residue suspended in dry dimethoxyethane (75 ml). A solution of sodium (2.42 g, 0.105 mole) in dry *n*-propanol (100 ml) was added all at once, and the mixture stirred for 1h at room temperature, and then heated under reflux for 16 hours. Solvents were removed *in vacuo*, and the residue treated with chloroform, the chloroform solution was washed with water (2 ×) and dried over $MgSO_4$. After removal of the solvent, the residue was subjected to column chromatography on silica gel, eluting with chloroform/ethyl acetate (4:1). The 4'-chlorophenylimino-C-(*n*-propoxy)methyl-3-pyridine was obtained in 58.5% yield as an oil.

*Analysis:*

| | |
|---|---|
| Calculated for $C_{15}H_{15}N_2OCl$: | C 65.6; H 5.5; N 10.2% |
| Found | : C 66.4; H 5.8; N 10.1% |

Example 4

*Preparation of 4'-chlorophenylimino-C-(n-butoxy)methyl-3-pyridine*

A stirred mixture of nicotinic-4'-chloroanilide (7 g, 0.03 mole) and thionyl chloride (22.5 ml) was heated under reflux for 2 hours. Excess thionyl chloride was removed *in vacuo*, and the residue suspended in dry dimethoxyethane (75 ml). A solution of sodium (2.42 g, 0.105 mole) in dry *n*-butanol (100 ml) was added all at once, and the mixture was stirred at room temperature for 1h, and then heated under reflux for 16 hours. Solvents were removed *in vacuo*, and the residue treated with chloroform; the chloroform solution was washed with water (2 ×) and dried over $MgSO_4$. After removal of the solvent, the residue was subjected to column chromatography on silica gel, eluting with ether/hexane (1:1). The 4'-chlorophenylimino-C-(*n*-butoxy)methyl-3-pyridine was obtained, after recrystallization from 40/60 petroleum spirit, in 46% yield as colourless crystals, melting point 53—4°C.

*Analysis:*

Calculated for $C_{16}H_{17}N_2OCl$: C 66.6; H 5.9; N 9.7%
Found : C 66.5; H 6.2; N 9.9%

Example 5

*Preparation of 4'-chlorophenylimino-C-(cyclohexyloxy)methyl-3-pyridine*

A mixture of nicotinic-4'-chloroanilide (7 g, 0.03 mole) and thionyl chloride (22.5 ml) was stirred and heated under reflux for 2 hours. Excess thionyl chloride was removed *in vacuo*, and the residue treated with dry pyridine (100 ml). To the stirred solution was added redistilled cyclohexanol (3.15 g, 5% excess), and subsequently the mixture was heated in an oil bath at 100°C for 6½ hours. After removal of the solvent *in vacuo*, the residue was treated with ether; the ethereal solution was washed with water (2 ×), and dried over $MgSO_4$. After removal of the solvent, the residue was subjected to column chromatography on silica gel, eluting with ether/hexane (1:1). The 4'-chlorophenylimino-C-(cyclohexyloxy)methyl-3-pyridine was obtained in 32% yield as a viscous oil.

*Analysis:*

Calculated for $C_{18}H_{19}N_2OCl$: C 68.7; H 6.0; N 8.9%
Found : C 67.8; H 6.2; N 8.7%

Example 6

*Preparation of 4'-chlorophenylimino-C-(methylthio)methyl-3-pyridine*

Nicontini-4'-chlorothianilide (2.485 g, 0.01 mole) was suspended in dry dimethoxyethane (40 ml). Sodium hydride (50% dispersion in oil — 0.48 g, 0.01 mole) was added, and the mixture stirred until a clear solution was obtained. Methyl iodide (1.42 g) was then added and the mixture stirred at ambient temperature for 19 hours. Solvent was removed *in vacuo*, and the residue treated with ether, the ethereal solution was washed with water (2 ×) and dried over $MgSO_4$. After removal of the solvent, the residue was subjected to column chromatography on silica gel, eluting with chloroform. The 4'-chlorophenylimino-C-(methylthio)methyl-3-pyridine was obtained in 97% yield as a solid, melting point 59—60.5°C.

*Analysis:*

Calculated for $C_{13}H_{11}N_2SCl$: C 59.4; H 4.2; N 10.7%
Found : C 59.1: H 4.5; N 10.7%

Example 7

*Preparation of 4'-chlorophenylimino-C-(tert.-butylthio)methyl-3-pyridine*

A stirred mixture of nicotinic-4'-chloroanilide (7 g, 0.03 mole) and thionyl chloride (22.5 ml) was heated under reflux for 2 hours. Excess thionyl chloride was removed *in vacuo*, and the residue treated with dry pyridine (100 ml). Tert.-butyl mercaptan (5.4 g, 0.06 mole) was added, and the mixture stirred and heated in an oil bath at 100—110°C for 16 hours. Solvent and volatiles were removed *in vacuo*, and the residue was treated with ether; the ethereal solution was washed with water (3 ×), and dried over $MgSO_4$. After removal of solvent, the residue was subjected to column chromatography on silica gel, eluting with $Et_2O$/hexane (1:1).

The 4'-chlorophenylimino-C-(*tert.*-butylthio)methyl-3-pyridine was obtained in 24% yield as a bright yellow crystalline solid, melting point 85—87°C.

*Analysis:*

Calculated for $C_{16}H_{17}N_2SCl$: C 63.05; H 5.58; N 9.20%
Found : C 62.8; H 5.7; N 9.1%

Example 8

*Preparation of 4'-chlorophenylimino-C-(carboxymethylthio)methyl-3-pyridine*

A stirred mixture of nicotinic-4'-chloroanilide (4.65 g. 0.02 mole) and thionyl chloride (20 ml) was heated under reflux for 2 hours. Excess thionyl chloride was removed *in vacuo*, and the residue treated with dry pyridine (50 ml). To this solution was added ethyl 2-mercaptoacetate (3.0 g, 0.025 mole) and the mixture was shaken for 3 hours at ambient temperature. Solvent was removed *in vacuo*, and the residue was treated with ether; the ethereal solution was washed with water and dried over $MgSO_4$. The crude ester, obtained by evaporation of the solvent was treated with NaOH (1.2 g, 0.03 mole) in $H_2O$ (10 ml) and methanol (35 ml), and the mixture was left for $2\frac{1}{2}$ hours at room temperature. Solvent was removed *in vacuo*, the residue treated with water (50 ml) and the aqueous solution extracted twice with ether. The aqueous phase was neutralized with acetic acid, to give an oil, which solidified. This was filtered off, and recrystallized from toluene, to give the product in 46% yield as an orange-brown solid, melting point 142—4°C.

*Analysis:*

| | |
|---|---|
| Calculated for $C_{14}H_{11}N_2SO_2Cl$ | : C 54.8; H 3.6; N 9.1: |
| Found | : 54.8; H 3.5; N 8.9% |

Example 9

*Preparation of 4'-chlorophenylimino-C-(*n*-butylamino)methyl-3-pyridine*

A stirred mixture of nicotinic-4'-chloroanilide (7 g, 0.03 mole) and thionyl chloride (22.5 ml was heated under reflux for 2 hours. Excess thionyl chloride was removed *in vacuo*, and the residue suspended in dry dimethoxyethane (75 ml). n-Butylamine (10.8 g 0.15 mole) was added, and the mixture was stirred at ambient temperature for 24 hours. Solvent was removed *in vacuo*, and the residue treated with ether; the ethereal solution was washed with water (2 ×) and dried over $MgSO_4$. After removal of solvent, the residue was subjected to column chromatography on silica gel, eluting with $Et_2O$/hexane (2:1). The 4'-chlorophenylimino-C-(*n*-butylamino)methyl-3-pyridine thus obtained, after recrystallization from 60/80 petroleum spirit, had a melting point of 65—67°C. The yield was 50%.

*Analysis:*

| | |
|---|---|
| Calculated for $C_{16}H_{18}N_3Cl$ | : C 66.78; H 6.26; N 14.61% |
| Found | : C 66.7; H 6.5; N 14.4% |

Example 10

*Preparation of 4'-chlorophenylimino-C-(di-*n*-butylamino)methyl-3-pyridine*

A stirred mixture of nicotinic-4'-chloroanilide (7 g, 0.03 mole) and thionyl chloride (22.5 ml) was heated under reflux for 2 hours. Excess thionyl chloride was removed *in vacuo*, and the residue suspended in dry dimethoxyethane (150 ml). Di-*n*-butylamine (19.4 g, 0.15 mole) was added, and the mixture was stirred for 2 hours at ambient temperature. The solvent was removed in vacuo, and the residue treated with ether; the ethereal solution was washed with water (2 ×) and dried over $MgSO_4$. After removal of the solvent, the residue was subjected to column chromatography on silica gel, eluting with ether/hexane (1:1). The 4'-chlorophenylimino-C-(di-*n*-butylamino)-methyl-3-pyridine was obtained in 27% yield as a viscous oil.

*Analysis:*

| | |
|---|---|
| Calculated for $C_{20}H_{26}N_3Cl$: | : C 69.9; H 7.6; N 12.2% |
| Found | : C 69.5; H 7.2; N 11.8% |

Example 11

*Preparation of 4'-chlorophenylimino-C-(N-piperidinyl)methyl-3-pyridine*

A stirred mixture of nicotinic-4'-chloroanilide (7 g, 0.03 mole) and thionyl chloride (22.5 ml) was heated under reflux for 2 hours. Excess thionyl chloride was removed *in vacuo*, and the residue suspended in dry dimethoxyethane (100 ml). Piperidine (9 g, 0.105 mole) was added, and the mixture stirred at ambient temperature for 16 hours.

Solvent was removed *in vacuo*, and the residue treated with ether; the ethereal solution was washed with water (2 ×) and dried over $MgSO_4$. After removal of solvent, the residue was subjected to column chromatography on silica gel, eluting with $Et_2O$/hexane (1:1). The 4'-chlorophenylimino-C-(N-piperidinyl)-methyl-3-pyridine was obtained in 11% yield as a viscous oil.

*Analysis:*

| | |
|---|---|
| Calculated for $C_{17}H_{18}N_3Cl$ | : C 68.11; H 6.01; N 14.02% |
| Found | : C 68.3; H 6.5; N 13.0% |

Examples 12—42

In an anlogous manner as described in the previous Examples the following compounds were prepared:

| Example No. | Compound | Analysis | |
|---|---|---|---|
| 12 | 4'-chlorophenylimino-C-(ethoxy)-methyl-3-pyridine | Calc. for $C_{14}H_{13}N_2OCl$ | : C 64.50 H 5.0 N 10.8 |
| | | Found | : C 64.9 H 5.1 N 10.5 |
| 13 | α-(4'-chlorophenylimino)ethyl-3-pyridine | Calc. for $C_{13}H_{11}N_2Cl$ | : C 67.7 H 4.8 N 12.1 |
| | | Found | : C 67.4 H 4.8 N 12.0 |
| 14 | 4'-chlorophenylimino-C-(p-fluoro-anilino)methyl-3-pyridine | Calc. for $C_{18}H_{13}N_3ClF$ | : C 66.4 H 4.0 N 12.9 |
| | | Found | : C 66.3 H 3.9 N 13.0 |
| 15 | 4'-chlorophenylimino-C-(methoxy)-methyl-3-pyridine | Calc. for $C_{13}H_{11}N_2OCl$ | : C 63.3 H 4.5 N 11.4 |
| | | Found | : C 63.6 H 4.6 N 11.7 |
| 16 | 4'-chlorophenylimino-C-(triazolyl)-methyl-3-pyridine | Calc. for $C_{14}H_{10}N_5Cl$ | : C 59.3 H 3.5 N 24.7 |
| | | Found | : C 59.4 H 3.4 N 23.9 |
| 17 | 4'-chlorophenylimino-C-(2-butoxy)-methyl-3-pyridine | Calc. For $C_{16}H_{17}N_2OCl$ | : C 66.6 H 5.9 N 9.7 |
| | | Found | : C 67.0 H 6.2 N 10.0 |
| 18 | 4'-chlorophenylimino-C-(isobutoxy)-methyl-3-pyridine | Calc. for $C_{16}H_{17}N_2OCl$ | : C 66.6 H 5.9 N 9.7 |
| | | Found | : C 66.2 H 6.2 N 9.6 |
| 19 | 4'-chlorophenylimino-C-(n-octyloxy)-methyl-3-pyridine | Calc. for $C_{20}H_{25}N_2OCl$ | : C 69.7 H 7.3 N 8.1 |
| | | Found | : C 69.7 H 7.6 N 8.4 |
| 20 | 4'-chlorophenylimino-C-(n-dodecyl)-oxy)methyl-3-pyridine | Calc. for $C_{24}H_{33}N_2OCl$ | : C 71.9 H 8.2 N 7.0 |
| | | Found | : C 71.8 H 8.2 N 7.0 |
| 21 | 4'-chlorophenylimino-C-(isopropyl-thio)methyl-3-pyridine | Calc. for $C_{15}H_{15}N_2SCl$ | : C 61.96 H 5.2 N 9.6 |
| | | Found | : C 61.8 H 5.2 N 9.6 |
| 22 | 4'-chlorophenylimino-C-(p-chloro-phenylthio)methyl-3-pyridine | Calc. for $C_{18}H_{12}N_2SCl_2$ | : C 60.2 H 3.3 N 7.8 |
| | | Found | : C 60.3 H 3.3 N 7.8 |
| 23 | 4'-chlorophenylimino-C-(di-n-propyl-amino)methyl-3-pyridine | Calc. for $C_{18}H_{22}N_3Cl$ | : C 68.5 H 7.0 N 13.3 |
| | | Found | : C 68.1 H 7.6 N 12.5 |
| 24 | 4'-chlorophenylimino-C-(n-nonyl-amino)methyl-3-pyridine | Calc. for $C_{21}H_{28}N_3Cl$ | : C 70.5 H 7.8 N 11.8 |
| | | Found | : C 70.5 H 7.9 N 11.5 |
| 25 | 4'-chlorophenylimino-C-(methoxy-ethoxy)methyl-3-pyridine | Calc. for $C_{15}H_{15}N_2O_2Cl$ | : C 62.0 H 5.2 N 9.6 |
| | | Found | : C 62.3 H 5.5 N 9.5 |

| Example No. | Compound | Analysis | |
|---|---|---|---|
| 26 | 2-chloro-4′-chlorophenylimino-C-(isopropoxy)methyl-3-pyridine | Calc. for $C_{15}H_{14}N_2OCl_2$ | : C 58.3 H 4.5 N 9.1 |
| | | Found | : C 58.0 H 4.4 N 8.9 |
| 27 | 4′-fluorophenylimino-C-(isopropoxy)-methyl-3-pyridine | Calc. for $C_{15}H_{15}N_2OF$ | : C 69.8 H 5.8 N 10.9 |
| | | Found | : C 69.7 H 5.8 N 10.7 |
| 28 | 3′,5′-dichlorophenylimino-C-(iso-propoxy)methyl-3-pyridine | Calc. for $C_{15}H_{14}N_2OCl_2$ | : C 58.3 H 4.5 N 9.1 |
| | | Found | : C 58.6 H 4.6 N 9.0 |
| 29 | 3′,5′-dichlorophenylimino-C-(iso-propylthio)methyl-3-pyridine | Calc. for $C_{15}H_{14}N_2SCl_2$ | : C 55.4 H 4.3 N 8.6 |
| | | Found | : C 55.7 H 4.4 N 8.6 |
| 30 | 2′,6′-dimethylphenylimino-C-(isp-propoxy)methyl-3-pyridine | Calc. for $C_{17}H_{20}N_2O$ | : C 76.1 H 7.5 N 10.5 |
| | | Found | : C 77.0 H 7.7 N 10.2 |
| 31 | 4′-fluorophenylimino-C-(isopropyl-thio)methyl-3-pyridine | Calc. for $C_{15}H_{15}N_2SF$ | : C 65.7 H 5.5 N 10.2 |
| | | Found | : C 66.0 H 5.7 N 10.4 |
| 32 | 3′,4′-dichlorophenylimino-C-(iso-propoxy)methyl-3-pyridine | Calc. for $C_{15}H_{14}N_2OCl_2$ | : C 58.3 H 4.5 N 9.1 |
| | | Found | : C 58.1 H 4.4 N 8.8 |
| 33 | Phenylimino-C-(isopropoxy)methyl-3-pyridine | Calc. for $C_{15}H_{16}N_2O$ | : C 75.0 H 6.7 N 11.7 |
| | | Found | : C 75.5 H 7.3 N 11.2 |
| 34 | 3′,4′-dichlorophenylimino-C-(iso-propylthio)methyl-3-pyridine | Calc. for $C_{15}H_{14}N_2SCl_2$ | : C 55.4 H 4.3 N 8.6 |
| | | Found | : C 56.1 H 4.5 N 8.7 |
| 35 | Phenylimino-C-(isopropylthio)-methyl-3-pyridine | Calc. for $C_{15}H_{16}N_2S$ | : C 70.3 H 6.2 N 11.0 |
| | | Found | : C 71.7 H 6.7 N 11.1 |
| 36 | 4′-bromophenylimino-C-(iso-propoxy)methyl-3-pyridine | Calc. for $C_{15}H_{15}N_2OBr$ | : C 56.4 H 4.7 N 8.8 |
| | | Found | : C 55.6 H 4.8 N 8.6 |
| 37 | 4′-bromophenylimino-C-(isopropyl-thio)methyl-3-pyridine | Calc. for $C_{15}H_{15}N_2SBr$ | : C 53.7 H 4.5 N 8.4 |
| | | Found | : C 53.5 H 4.5 N 8.3 |
| 38 | 2′,4′-dichlorophenylimino-C-(iso-propoxy)methyl-3-pyridine | Calc. for $C_{15}H_{14}N_2OCl_2$ | : C 58.3 H 4.5 N 9.1 |
| | | Found | : C 58.0 H 4.5 N 8.9 |
| 39 | 2-isopropoxy-4′-chlorophenylimino-C-(isopropoxy)methyl-3-pyridine | Calc. for $C_{18}H_{21}N_2O_2Cl$ | : C 65.0 H 6.3 N 8.4 |
| | | Found | : C 64.3 H 6.0 N 7.9 |
| 40 | 3′-chloro-4′-fluorophenylimino-C-(isopropylthio)methyl-3-pyridine | Calc. for $C_{15}H_{14}N_2SClF$ | : C 58.4 H 4.5 N 9.1 |
| | | Found | : C 58.6 H 4.9 N 8.9 |

| Example No. | Compound | Analysis | |
|---|---|---|---|
| 41 | 2′,4′-dichlorophenylimino-C-(isopropylthio)methyl-3-pyridine | Calc. for $C_{15}H_{14}N_2SCl_2$ | : C 55.4 H 4.3 N 8.6 |
| | | Found | : C 56.1 H 4.6 N 8.5 |
| 42 | 2-chloro-4′-chlorophenylimino-C (isopropylthio)methyl-3-pyridine | Calc. for $C_{15}H_{14}N_2SCl_2$ | : C 55.4 H 4.3 N 8.6 |
| | | Found | : C 55.5 H 4.2 N 8.4 |
| 43 | 4′-chlorophenylimino-C-(diisopropylaminoethylamino)methyl-3-pyridine | Calc. for $C_{20}H_{27}N_4Cl$ | : C 67.0 H 7.5 N 15.6 |
| | | Found | : C 67.0 H 7.5 N 15.5 |
| 44 | 4′-chlorophenylimino-C-(di-3-methylbutylamino)methyl-3-pyridine | Calc. for $C_{22}H_{30}N_3Cl$ | : C 71.1 H 8.1 N 11.3 |
| | | Found | : C 71.4 H 8.4 N 11.1 |
| 45 | 4′-chlorophenylimino-C-(methoxyethylamino)methyl-3-pyridine | Calc. for $C_{15}H_{16}N_3OCl$ | : C 62.2 H 5.5 N 14.5 |
| | | Found | : C 62.2 H 5.5 N 14.6 |
| 46 | 4′-chlorophenylimino-C-(*n*-hexadecylamino)methyl-3-pyridine | Calc. for $C_{28}H_{42}N_3Cl$ | : C 73.8 H 9.2 N 9.2 |
| | | Found | : C 74.1 H 9.9 N 8.9 |
| 47 | 4′-chlorophenylimino-C-(hydroxyethylamino)methyl-3-pyridine | Calc. for $C_{14}H_{14}N_3OCl$ | : C 61.0 H 5.1 N 15.3 |
| | | Found | : C 61.0 H 5.2 N 15.2 |
| 48 | 4′-chlorophenylimino-C-(N-pyrolidinyl)methyl-3-pyridine | Calc. For $C_{16}H_{16}N_3Cl$ | : C 67.3 H 5.6 N 14.7 |
| | | Found | : C 67.4 H 5.7 N 14.8 |
| 49 | 4′-chlorophenylimino-C-(benzylamino)methyl-3-pyridine | Calc. for $C_{19}H_{16}N_3Cl$ | : C 70.9 H 5.0 N 13.1 |
| | | Found | : C 70.6 H 5.0 N 12.9 |
| 50 | 2-chloro-4′-chlorophenylimino-C-(isopropoxy)methyl-3-pyridine | Calc. for $C_{15}H_{14}N_2OCl_2$ | : C 58.3 H 4.5 N 9.1 |
| | | Found | : C 58.3 H 4.6 N 9.0 |
| 51 | 3′-chlorophenylimino-C-(isopropoxy)methyl-3-pyridine | Calc. for $C_{15}H_{15}N_2OCl$ | : C 65.6 H 5.5 N 9.8 |
| | | Found | : C 66.6 H 5.9 N 10.2 |
| 52 | α-(4′-chlorophenylimino)ethyl 4-pyridine | Calc. for $C_{13}H_{11}N_2Cl$ | : C 67.7 H 4.8 N 12.1 |
| | | Found | : C 67.5 H 4.8 N 12.0 |
| 53 | α-(4′-chlorophenylimino)ethyl-2-pyridine | Calc. for $C_{13}H_{11}N_2Cl$ | : C 67.7 H 4.8 N 12.1 |
| | | Found | : C 67.3 H 4.7 N 11.6 |
| 54 | 4′-chlorophenylimino-C-(isopropoxy-methyl-4-pyridine | Calc. for $C_{15}H_{15}N_2OCl$ | : C 65.6 H 5.5 N 10.2 |
| | | Found | : C 65.4 H 5.7 N 10.0 |
| 55 | 2′,4′-dichlorophenylimino-C-(isopropoxy)methyl-4-pyridine | Calc. for $C_{15}H_{14}N_2OCl_2$ | : C 58.3 H 4.5 N 9.1 |
| | | Found | : C 59.0 H 4.3 N 8.4 |

| Example No. | Compound | Analysis | |
|---|---|---|---|
| 56 | 3′,4′-dichlorophenylimino-C-(isopropoxy)methyl-4-pyridine | Calc. for $C_{15}H_{14}N_2OCl_2$ | : C 58.3 H 4.5 N 9.1 |
| | | Found | : C 58.1 H 4.7 N 8.8 |
| 57 | 2′,6′-dichlorophenylimino-C-(isopropoxy)methyl-4-pyridine | Calc. for $C_{15}H_{14}N_2OCl_2$ | : C 58.3 H 4.5 N 9.1 |
| | | Found | : C 57.9 H 4.8 N 8.4 |
| 58 | 2′,5′-dichlorophenylimino-C-(isopropoxy)methyl-4-pyridine | Calc. for $C_{15}H_{14}N_2OCl_2$ | : C 58.3 H 4.5 N 9.1 |
| | | Found | : C 57.5 H 4.6 N 8.5 |
| 59 | 2′,3′-dichlorophenylimino-C-(isopropoxy)methyl-4-pyridine | Calc. for $C_{15}H_{14}N_2OCl_2$ | : C 58.3 H 4.5 N 9.1 |
| | | Found | : C 58.3 H 4.6 N 9.0 |
| 60 | 3′,5′-dichlorophenylimino-C-(isopropoxy)methyl-4-pyridine | Calc. for $C_{15}H_{14}N_2OCl_2$ | : C 58.3 H 4.5 N 9.1 |
| | | Found | : C 58.3 H 4.6 N 9.0 |
| 61 | 4′-chlorophenylimino-C-(cyano)-methyl-4-pyridine | Calc. for $C_{13}H_8N_3Cl$ | : C 64.6 H 3.3 N 17.4 |
| | | Found | : C 64.8 H 3.3 N 17.3 |
| 62 | 3′-chloro-4′-fluorophenylimino-C-(isopropoxy)methyl-4-pyridine | Calc. for $C_{15}H_{14}N_2OClF$ | : C 61.5 H 4.8 N 9.6 |
| | | Found | : C 61.6 H 4.9 N 9.4 |
| 63 | 4′-chlorophenylimino-C-(N-morpholinyl)methyl-4-pyridine | Calc. for $C_{16}H_{12}N_3OCl$ | : C 63.6 H 5.3 N 13.9 |
| | | Found | : C 63.8 H 5.2 N 14.0 |
| 64 | 4′-chlorophenylimino-C-(di-2-methylpropylamino)methyl-4-pyridine | Calc. for $C_{20}H_{26}N_3Cl$ | : C 69.9 H 7.6 N 12.2 |
| | | Found | : C 71.2 H 8,1 N 12.3 |
| 65 | 4′-chlorophenylimino-C-(3,5-dimethylpiperidin-1-yl)methyl-3-pyridine | Calc. for $C_{19}H_{22}N_3Cl$ | : C 69.62 H 6.72 N 12.82 |
| | | Found | : C 69.5 H 6.9 N 12.9 |
| 66 | 2′,3′-dichlorophenylimino-C-(isopropoxy)methyl-3-pyridine | Calc. for $C_{15}H_{14}N_2OCl_2$ | : C 58.25 H 4.53 N 9.06 |
| | | Found | : C 57.9 H 4.6 N 8.9 |
| 67 | 2′-chlorophenylimino-C-(n-butoxy)-methyl-3-pyridine | Calc. for $C_{16}H_{17}N_2OCl$ | : C 66.6 H 5.9 N 9.7 |
| | | Found | : C 66.5 H 6.1 N 9.6 |
| 68 | 4′-methylphenylimino-C-(n-butoxy)-methyl-3-pyridine | Calc. for $C_{17}H_{20}N_2O$ | : C 76.1 H 7.5 N 10.4 |
| | | Found | : C 76.5 H 7.7 N 10.4 |
| 69 | 3′-methylphenylimino-C-(n-butoxy)-methyl-3-pyridine | Calc. for $C_{17}H_{20}N_2O$ | : C 76.1 H 7.5 N 10.4 |
| | | Found | : C 75.3 H 7.7 N 10.4 |
| 70 | 2′-methylphenylimino-C-(n-butoxy)-methyl-3-pyridine | Calc. for $C_{17}H_{20}N_2O$ | : C 76.1 H 7.5 N 10.4 |
| | | Found | : C 76.1 H 8.0 N 10.0 |

| Example No. | Compound | Analysis | |
|---|---|---|---|
| 71 | 2′,3′-dichlorophenylimino-C-(n-butoxy)-methyl-3-pyridine | Calc. for $C_{16}H_{16}N_2OCl_2$ | : C 59.63 H 4.97 N 8.7 |
| | | Found | : C 58.5 H 5.0 N 8.4 |
| 72 | 4′-methylphenylimino-C-(iso-propylthio)methyl-3-pyridine | Calc. for $C_{16}H_{18}N_2S$ | : C 71.1 H 6.67 N 10.37 |
| | | Found | : C 70.7 H 6.9 N 10.4 |
| 73 | 2′,5′-dichlorophenylimino-C-(iso-propylthio)methyl-3-pyridine | Calc. for $C_{15}H_{14}N_2SCl_2$ | : C 55.4 H 4.31 N 8.62 |
| | | Found | : C 55.2 H 4.4 N 8.5 |
| 74 | 2′,6′-dichlorophenylimino-C-(n-butoxy)methyl-3-pyridine | Calc. for $C_{16}H_{16}N_2OCl_2$ | : C 59.44 H 4.95 N 8.7 |
| | | Found | : C 59.7 H 5.2 N 8.6 |
| 75 | 2′,6′-dichlorophenylimino-C-(iso-propylthio)-methyl-3-pyridine | Calc. for $C_{15}H_{14}N_2SCl_2$ | : C 55.4 H 4.31 N 8.62 |
| | | Found | : C 55.0 H 4.1 N 9.3 |
| 76 | 2′-chloro-4′-methylphenylimino-C-(n-butoxy)methyl-3-pyridine | Calc. for $C_{17}H_{19}N_2OCl$ | : C 67.44 H 6.28 N 9.26 |
| | | Found | : C 67.5 H 6.5 N 9.6 |
| 77 | 2′-chloro-4′-methylphenylimino-C-(isopropylthio)methyl-3-pyridine | Calc. for $C_{16}H_{17}N_2SCl$ | : C 63.05 H 5.58 N 9.2 |
| | | Found | : C 63.4 H 5.9 N 9.2 |
| 78 | 3′-chloro-4′-methylphenylimino-C-(n-butoxy)methyl-3-pyridine | Calc. for $C_{17}H_{19}N_2OCl$ | : C 67.44 H 6.28 N 9.26 |
| | | Found | : C 67.4 H 6.4 N 9.1 |
| 79 | 3′-chloro-4′-methylphenylimino-C-(isopropylthio)methyl-3-pyridine | Calc. for $C_{16}H_{17}N_2SCl$ | : C 63.05 H 5.58 N 9.2 |
| | | Found | : C 63.1 H 5.8 N 9.0 |
| 80 | 2′-methyl-4′-chlorophenylimino-C-(n-butoxy)methyl-3-pyridine | Calc. for $C_{17}H_{19}N_2OCl$ | : C 67.44 H 6.28 N 9.26 |
| | | Found | : C 67.3 H 6.5 N 9.0 |
| 81 | 2′-methyl-4′-chlorophenylimino-C-(isopropylthio)methyl-3-pyridine | Calc. For $C_{16}H_{17}N_2SCl$ | : C 63.05 H 5.58 N 9.2 |
| | | Found | : C 63.1 H 5.8 N 9.0 |
| 82 | 2′-chloro-5′-methylphenylimino-C-(n-butoxy)methyl-3-pyridine | Calc. for $C_{17}H_{19}N_2OCl$ | : C 67.44 H 6.28 N 9.26 |
| | | Found | : C 67.4 H 6.5 N 9.1 |
| 83 | 3′-methyl-4′-bromophenylimino-C-(n-butoxy)methyl-3-pyridine | Calc. for $C_{17}H_{19}N_2OBr$ | : C 58.81 H 5.48 N 8.07 |
| | | Found | : C 58.5 H 5.6 N 7.8 |
| 84 | 4′-fluorophenyliminomethyl-3-pyridine | Calc. for $C_{12}H_9N_2F$ | : C 72.0 H 4.5 N 14.0 |
| | | Found | : C 72.6 H 4.8 N 14.1 |

Example 85

The funigical activity of the compounds was investigated by means of the following tests:

(a) *Activity against vine downy mildew (Plasmopera viticola Pv.a)*

The test is a direct anit-sproulant one using a foliar spray. The lower surfaces of leaves of whole vine plants, are inoculated by spraying with an aqueous suspension containing $10^5$ zoosporangia/ml 4 days

prior to treatment with the test compound. The inoculated plants are kept for 24 hours in a high humidity compartment, 48 hours at glasshouse ambient temperature and humidity and then returned for a further 24 hours to high humidity. The plants are then dried and infected leaves detached and sprayed on the lower surfaces at a dosage of 1 kilogram of active material per hectare using a track sprayer. After drying the petioles of the sprayed leaves are dipped in water and the leaves returned to high humidity for a further 72 hours incubation, followed by assessment. Assessment is based on the percentage of the leaf area covered by sporulation compared with that on control leaves.

b) *Activity against vine downy mildew (Plasmopera viticola Pv.T)*

The test is a translaminar protectant one using a foliar spray. The upper surfaces of leaves of whole vine plants are sprayed at a dosage of 1 kilogram of active material per hectare using a track sprayer. The lower surfaces of the leaves are then inoculated, up to 6 hours after treatment with the test compound, by spraying with an aqueous suspension containing $10^5$ zoosporangia/ml. The inoculated plants are kept for 24 hours in a high humidity compartment, 4 days at glasshouse ambient temperature and humidity and then returned for a further 24 hours to high humidity. Assessment is based on the percentage of the leaf area covered by sporulation compared with that on control leaves.

c) *Activity against vine grey mould (botrytis cinerea B.c.)*

The test is a direct eradicant one using a foliar spray. The under-surfaces of detached vine leaves are inoculated by pipetting ten large drops of an aqueous suspension containing $5 \times 10^5$ conidia/ml on to them. The inoculated leaves are kept uncovered overnight during which time the drops containing the conidia slowly dry. By this time the fungus has penetrated the leaf and a visible necrotic lesion may be apparent where the drop was made. The infected regions are sprayed directly with a dosage of 1 kg of active material per hectare using a track sprayer. When the spray has dried the leaves are covered with petri dish lids and the disease allowed do develop under the moist conditions. The extent of the necrotic lesion beyond the original drop together with the degree of sporulation is compared with that on control plants.

d) *Activity against potato late blight (Phytophthorainfestans* P.i.e.)

The test is a direct eradicant one using a foliar spray. The upper surfaces of the leaves of potato plants (12—18 cms high, in monopots) are inoculated by spraying with an aqueous suspension containing $5 \times 10^3$ zoosporangia/ml 16—19 hours prior to treatment with the test compound. The inoculated plants are kept overnight at high humidity and then allowed to dry before spraying at a dosage of 1 kg of active material per hectare using a track sprayer. After spraying the plants are returned to high humidity for a further period of 48 hours. Assessment is based on a comparison between the levels of disease on the treated and control plants.

e) *Activity against barley powdery mildew (Erysiphe graminis Eg.)*

The test measures the direct anti-sporulant activity of compounds applied as a foliar spray. For each compound about 40 barley seedlings were grown to the one-leaf stage in a plastic pot of sterile potting compost. Inoculation was effected by dusting the leaves with conidia of Erysiphe graminis, spp. hordei. 24 hours after inoculation the seedlings were sprayed with a solution of the compound in a mixture of acetone (50%), surfactant (0.04%) and water using a track sprayer. The rate of application was equivalent to 1 kg of active material per hectare. First assessment of disease was made 5 days after treatment, when the overall level of sporulation of the treated pots were compared with that on control pots.

f) *Activity against wheat brown rust (puccinia recondita* P.r)

The test is a direct antisporulant one using a foliar spray. Pots containing about 25 wheat seedlings per pot, at first leaf stage, were inoculated by spraying the leaves with an aqueous suspension, containing $10^5$ spores/ml plus a little Triton X—155, 20—24 hours before treatment with the compound under test. The inoculated plants were kept overnight in a high humidity compartment, dried at glass-house ambient temperature and then sprayed at a dosage of 1 kilogram of active material per hectare using a track-sprayer. After treatment the plants were kept at glasshouse ambient temperature and assessment made about 11 days after treatment. Assessment is based on the relative density of sporulating pustules per plant compared with that on control plants.

g) *Activity against broad bean rust (Uromyces fabae* U.f)

The test is a translaminar antisporulant one using foliar spray. Pots containing 1 plant per pot were inoculated by spraying an aqueous suspension, containing $5 = 10^4$ spores/ml plus a little Triton X—155, onto the undersurface of each leaf 20—24 hours before treatment with test compound. The inoculated plants were kept overnight in a high himidity compartment, dried at glass-house ambient temperature and then sprayed, on the leaf upper surface, at a dosage of 1 kg/ha of active material using a track sprayer. After treatment the plants were kept at glass-house temperature and assessment made

11—14 days after treatment. Symptoms are assessed on the relative density of sporulating pustules per plant compared with that on control plants.

h) *Activity against rice leaf blast (Pyricularia oryzae* P.o*)*

The test is a direct eradicant one using a foliar spray. The leaves of rice seedlings (about 30 seedlings per pot) are sprayed with an aqueous suspension containing 105 sports/ml 20—24 hours prior to treatment with the test compound. The inoculated plants are kept overnight in high humidity and then allowed to dry before spraying at a dosage of 1 kg of active material per hectare using a track sprayer. After treatment the plants are kept in a rice compartment at 25—30°C and high humidity. Assessments are made 4—5 days after treatment and are based on the density of necrotic lesions and the degree of withering when compared with control plants.

i) *Activity against rice sheath blight (Pellicularia sasakii* P.s.*)*

The test is a direct eradicant one using a foliar spray. 24—24 hours prior to treatment with the test compound rice seedlings (about 30 seedlings per pot) are sprayed with 5 ml of an aqueous suspension containing 0.2 g of crushed sclerotia/myelium per ml. The inoculated plants are kept overnight in a humid cabinet maintained at 25—30°C, followed by spraying at a dosage of 1 kg of active material per hectare. The treated plants are then returned to high humidity for a further period of 3—4 days. With this disease brown leasions are seen that start at the base of the sheath and extend upwards. Assessments are made on the number and extent of the lesions when compared with the control.

j) *Activity against potato late blight (Phytophthora infestans P.i.p)*

The test measures the direct protectant activity of compounds applies as a foliar spray. Tomato plants, Cultivar Ailsa Craig, 1—15 cs high, in monopots are used. The whole plant is sprayed at a dosage of 1 kilogram of active material per hectare using a track sprayer. The plant is then inoculated up to 6 hours after treatment with the test compound, by spraying with an aqueous suspension containing 5 x $10^3$ zoosporangia/ml. The inoculated plants are kept in high humidity for 3 days. Assessment is based on a comparison between the levels of disease on the treated and control plants.

The extent of disease control is expressed as a control rating according to the criteria:

0 = less than 50% disease control

1 = 50—80% disease control

2 = greater than 80% disease control

/S1 and /S2 indicated systemic activity, using the same scale of rating

The obtained control ratings are set out in Table I:

TABLE I

| Example No. | Pv a | Pv t | Bc | Pi e | Pi p | Eg | Pr | Uf | Po | Ps |
|---|---|---|---|---|---|---|---|---|---|---|
| 1 | 2 | | | | | 2 | 2 | | | |
| 2 | | | | | / S2 | 2/ S2 | | | | |
| 3 | 1 | 2 | | | | 2 | | | | 2 |
| 4 | 2 | 2 | | | | 2 | 1 | | | |
| 5 | | | | | | 2 | | | | 2 |
| 6 | 2 | | | | | 2 | 1 | | | |
| 7 | | | | | | 2 | | 1 | 1 | 1 |
| 8 | | | | | | 0 | | | | |
| 9 | | | | | | 2 | | | | |
| 10 | | | | | | 2 | | | | 2 |
| 11 | | 2 | | 2 | | 2 | | | | |
| 12 | | 2 | 2 | | | 2 | | | | |
| 13 | | | | | | 1 | | | | |
| 14 | 1 | | | | | 0 | | | | |
| 15 | 2 | | | 1 | | 2 | | | | |
| 16 | | 2 | | | | 1 | | | | |
| 17 | | | | | | 2/ S1 | | | | |
| 18 | | | | | | 2 | | | | 1 |
| 19 | | | | | | 2 | | | | |
| 20 | 1 | 1 | | | / S1 | 2 | | | 1 | |
| 21 | | | | | | 2 | | | | |
| 22 | | | | | | 1 | | | | |
| 23 | 1 | | | | | 2/ S2 | | | | |
| 24 | | | 2 | | | | | | | |
| 25 | | 1 | | | / S1 | 2 | | | | |
| 26 | | 1 | | | | | | | | |
| 27 | | | | | | 2 | | | 1 | |
| 28 | | | | | | 2 | | | | |

TABLE I (Continued)

| Example No. | Pv a | Pv t | Bc | Pi e | Pi p | Eg | Pr | Uf | Po | Ps |
|---|---|---|---|---|---|---|---|---|---|---|
| 29 | | | | | | 2 | | | | |
| 30 | | | | | | 1 | | | | |
| 31 | | | | | | 2 | | | | |
| 32 | | | | | | 2 | | | | |
| 33 | 1 | | | | | 1 | | | | |
| 34 | | | | | | 2 | | | 1 | |
| 35 | | | | | | 2 | | | 1 | |
| 36 | | | | | | 2 | | | | |
| 37 | | 2 | | | | 2 | | | | |
| 38 | | | 1 | | | 2 | | | | |
| 39 | | 1 | | | | | | | | |
| 40 | | | | | | 2 | | | | |
| 41 | | | | | | 2 | | | | |
| 42 | | | | | | 2 | | | | |
| 43 | 1 | | | | | 1 | | | | |
| 44 | | | | | | 0 | | | | |
| 45 | | | | | | 1 | | | | |
| 46 | | | | | | 0 | | | | |
| 47 | | | | | | - | | | | |
| 48 | | | | | | - | | | | |
| 49 | | | | | | - | | | | |
| 50 | | | | | | 0 | | 1 | | |
| 51 | | | | | | - | | | | |
| 52 | | | | | | - | | | | |
| 53 | | | | | | - | | | | |
| 54 | | | | | | 2 | 1 | | | |
| 55 | | | | | | 2 | | | | |
| 56 | | | | | | 2 | | | | |

TABLE I (Continued)

| Example No. | Pv a | Pv t | Bc | Pi e | Pi p | Eg | Pr | Uf | Po | Ps |
|---|---|---|---|---|---|---|---|---|---|---|
| 57 | | | | | | 0 | | | | |
| 58 | | | | | | 0 | | | 1 | |
| 59 | | 1 | | | | 0 | | | 2 | |
| 60 | | | | | | 0 | | | | |
| 61 | | | | | | 0 | 2 | | | |
| 62 | | | | | | 0 | | | | |
| 63 | | | | | /S1 | 0 | | | | |
| 64 | | 1 | | | | 2 | 2 | | | |
| 65 | | | | | | 2 | | | | |
| 66 | | | | | | 2 | | | | |
| 67 | | | | | | 2 | | | | |
| 68 | | | | | | 2 | | | | |
| 69 | | | | | | 2 | | | | |
| 70 | | | | | | 2 | | | | |
| 71 | | | | | | 2 | | | | |
| 72 | | | | | | 0 | | | | |
| 73 | | | | | | 2 | 1 | | | |
| 74 | | | | | | 2 | | | | |
| 75 | | | | | | 2 | | | | |
| 76 | | | | | | 2 | | | | |
| 77 | | | | | | 2 | | | | |
| 78 | 1 | | | | | 2 | | | | |
| 79 | | | | | | 2 | | | | |
| 80 | | | | | | 2 | | | | |
| 81 | | | | | | 2 | | | | |
| 82 | | | | | | 0 | | | | |
| 83 | | | | | | 0 | | | | |
| 84 | | | | | | – | | | | |

Example 86

The plant growth regulating properties of various compounds were investigated with the aid of the following test:

The plants used in the evaluation of the compounds were determinate soya bean cultivars (e.g. Fiskeby V).

Liquid formulations of the compounds were applied to plants at an early stage of flower development. The formulations used consisted of 60% water and 40% acetone which contained 0.4% TRITON X155, and amounts of the test compound to give a spray application equivalent to 0.5, 1.0 and 2.0 kg/ha.

The soya bean seeds were inoculated with a commercial strain of *Rhizobium* ("Nitrogerm", Root Nodule Pty., Ltd., Australia) prior to sowing in a loam:grit (5:1) mixture in 5" diameter pots. Plants were maintained at 21—25°C under 14 hr. daylength and watering by sub-irrigation. Treatments were as foliar applications to three plants for each dose of the test material, applied in a volume equivalent to 632 litres per hectare using a fixed 'jaw' nozzle. After treatment the plants were set out in a randomised block design. Height (cm), phytotoxicity and other physiological effects were recorded one week after treatment. After a further three weeks the total pod number per plant was recorded. Results were derandomised and mean values calculated and expressed as a percentage of the untreated controls. The pod numbers thus obtained are given in Table II.

TABLE II

| Compound of \ Dose kg/ha | 0.5 | 1.0 | 2.0 |
|---|---|---|---|
| Example 12 | 89 | 115 | 121 |
| ,, 13 | 118 | 136 | 105 |
| ,, 15 | 138 | 161 | 138 |
| ,, 3 | 100 | 131 | 138 |
| ,, 4 | 123 | 146 | 154 |
| ,, 17 | 146 | 115 | – |
| ,, 18 | 115 | 131 | 146 |
| ,, 19 | 123 | 146 | 138 |
| ,, 21 | 138 | 146 | 169 |
| ,, 29 | 113 | 117 | 117 |
| ,, 32 | 121 | 121 | 132 |
| ,, 37 | 129 | 138 | 125 |
| ,, 25 | 103 | 127 | – |
| ,, 50 | 103 | 112 | 118 |

Example 87

In a test, carried out to investigate the abscission activity of various compounds, the following procedure was applied:

French bean (cv. Canadian Wonder) were used as the indicator species for abscission activity. French bean seeds were sown at the rate of 2 per 8 cm pot in sterilized loam. Plants were maintained at 20°C under 14 hr daylength and watered by sub irrigation. At the first trifoliate leaf stage of development, the laminae of the primary leaves were removed. 48 hrs. after removal of the laminae, liquid formulations of the test compounds were applied. The formulation used, consisted of 90% water

and 10% acetone which contained 0.4% TRITON X155 and amounts of the test compound to give spray application of 50, 250, 1000, and 2000 ppm.

Treatments were as foliar applications to "run off" using a fixed nozzle. After treatment the plants were set out in randomised block design.

The number of petioles which had abscissed from the main stem were recorded three, five and ten days after treatment. Phytotoxicity and any other physiological effects were also recorded 10 days after treatment. Results were derandomised and the mean value of the tree replicates per treatment calculated.

The obtained results are given in Table III as either no abscission activity (0), or greater than 75% activity (+) as compared with untreated control.

TABLE III

| Compound of \ Dose ppm | 50 | 250 | 1000 | 2000 |
|---|---|---|---|---|
| Example 19 | 0 | 0 | – | + |
| ,, 20 | 0 | 0 | – | + |
| ,, 21 | + | – | + | – |
| ,, 36 | 0 | 0 | + | + |
| ,, 37 | 0 | 0 | + | + |
| ,, 41 | 0 | 0 | 0 | + |
| ,, 67 | 0 | 0 | + | + |
| ,, 68 | 0 | + | + | + |
| ,, 70 | 0 | 0 | 0 | + |

**Claims for the Contracting States: BE CH DE FR GB IT LU NL**

1. Use as a fungicide and/or a plant growth regulant of a compound of formula:

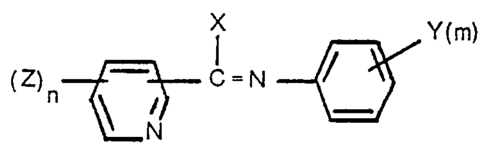

(I)

wherein X represents a hydrogen atom, a cyano group, a non-substituted or a halo- or alkoxy-substituted alkoxy-group, a cycloalkloxy group, an alkylthio, carboxymethylthio, phenylthio, 2-chlorophenylthio, 4-chlorophenylthio, 4-bromophenylthio, 2,4-dichlorophenylthio, an alkyl group or a group NRR′, in which R is a hydrogen atom or an alkyl group and R′ is an alkyl, a phenyl or a cycloalkyl group and when R is hydrogen R′ can be p-fluorophenyl, benzyl, diisopropylamino ethyl, methoxy ethyl or hydroxy ethyl or R and R′ together with the interjacent nitrogen atom may form a heterocyclic ring chosen from triazole, pyrolidine, morpholine, piperidine, or 3,5-dimethylpiperidine, Y and Z independently are halogen atom, alkyl-, alkoxy- or nitro groups and m and n are each 0, 1 or 2 whereby the same or different groups Y may be present when m equals 2 and the same or different groups Z when n equals 2.

2. Use as claimed in claim 1 of a compound of formula I which is a 3-pyridyl derivate.

3. Use as claimed in claim 1 or 2 of a compound of formula I wherein X represents an alkoxy group.

4. Use as claimed in claim 1 or 2 of a compound of formula I wherein X represents an alkylamino group.

5. Use as claimed in any of claims 1 to 4 of a compound of formula I wherein Y represents a halogen atom.

6. Use as claimed in claim 1 of 4′-chlorophenylimino-C-(cyano)methyl-3-pyridine.
7. Use as claimed in claim 1 of 4′-chlorophenylimino-C-(n-propoxy)methyl-3-pyridine.
8. Use as claimed in claim 1 of 4′-chlorophenylimino-C-(n-butoxy)methyl-3-pyridine.
9. Use as claimed in claim 1 of 4′-chlorophenylimino-C-(cyclo-hexyloxy)methyl-3-pyridine.
10. Use as claimed in claim 1 of 4′-chlorophenylimino-C-(methylthio)methyl-3-pyridine.
11. Use as claimed in claim 1 of 4′-chlorophenylimino-C-(tert.butylthio)methyl-3-pyridine.
12. Use as claimed in claim 1 of 4′-chlorophenylimino-C-(di-n-butylamino)methyl-3-pyridine.
13. Use as claimed in claim 1 of 4′-chlorophenylimino-C-(N-piperidinyl)methyl-3-pyridine.
14. A pesticidal and/or plant growth regulating composition comprising a surface active agent and optionally a carrier or a solid carrier and as pesticidal and/or plant growth regulating ingredient at least one compound of formula I in claim 1 as defined in any of claims 1 to 13, provided that when X is hydrogen, n is 0 or Z is methyl and n is 1, m = 1 and the derivative is a 2- or 4- pyridyl derivative then Y is not methyl, methoxy, ethoxy or chloro; and when X is hydrogen and the derivative is a 6-methyl-2-pyridyl (n = 1) derivative or a 4-pyridyl (n = 0) derivative then m is not 0.
15. Phenyliminomethylpyridine derivatives of formula I in claim 1 as defined in claim 14, provided that when X is hydrogen, and n = 0, then m is not 0; and when X is hydrogen and n = 0 then m is not 1 when Y is chloro or methoxy; and when X is hydrogen the derivative is a 3- or 4-pyridyl derivative, and m = 1, then Y is not a 3-nitro substituent; and when X is a cyano group, the derivative is a 4-pyridyl derivative, and n = 0 and m = 1, then Y is other than a 4-methoxy substituent.
16. A process for the preparation of compounds of formula I in claim 1 other than those in which X is hydrogen or alkyl, as defined in claim 2, in which a nicotinic anilide is reacted with a thionyl halide, and the formed phenylimino-C-(halo)methyl-3-pyridine is further converted by reaction with a compound QX, X having the aforesaid meaning with the exception of hydrogen and alkyl and Q being hydrogen, an alkali metal or copper.
17. A process as claimed in claim 16 wherein Q represents hydrogen or an alkali metal.
18. A process as claimed in claim 16 or 17 wherein the thionyl halide is thionyl chloride.

**Claims for the Contracting State: AT**

1. Use as a fungicide and/or a plant growth regulant of a compound of formula:

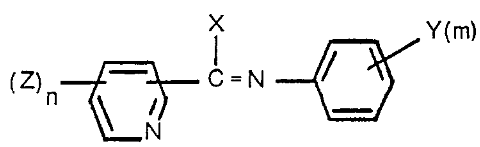

(I)

wherein X represents a hydrogen atom, a cyano group, a non-substituted or a halo- or alkoxy-substituted alkoxy-group, a cycloalkloxy group, an alkylthio, carboxymethylthio, phenylthio, 2-chlorophenylthio, 4-chlorophenylthio, 4-bromophenylthio, 2,4-dichlorophenylthio, an alkyl group or a group NRR′, in which R is a hydrogen atom or an alkyl group and R′ is an alkyl, a phenyl or a cycloalkyl group and when R is hydrogen R′ can be p-fluorophenyl, benzyl, diisopropylamino ethyl, methoxy ethyl or hydroxy ethyl or R and R′ together with the interjacent nitrogen atom may form a heterocyclic ring chosen from triazole, pyrolidine, morpholine, piperidine, or 3,5-dimethylpiperidine, Y and Z independently are halogen atom, alkyl-, alkoxy- or nitro groups and m and n are each 0, 1 or 2, whereby the same or different groups Y may be present when m equals 2 and the same or different groups Z when n equals 2.
2. Use as claimed in claim 1 of a compound of formula I which is a 3-pyridyl derivative.
3. Use as claimed in claim 1 or 2 of a compound of formula I wherein X represents an alkoxy group.
4. Use as claimed in claim 1 or 2 of a compound of formula I wherein X represents an alkylamino group.
5. Use as claimed in any of claims 1 to 4 of a compound of formula I wherein Y represents a halogen atom.
6. Use as claimed in claim 1 of 4′-chlorophenylimino-C-(cyano)methyl-3-pyridine.
7. Use as claimed in claim 1 of 4′-chlorophenylimino-C-(n-propoxy)methyl-3-pyridine.
8. Use as claimed in claim 1 of 4′-chlorophenylimino-C-(n-butoxy)methyl-3-pyridine.
9. Use as claimed in claim 1 of 4′-chlorophenylimino-C-(cyclo-hexyloxy)methyl-3-pyridine.
10. Use as claimed in claim 1 of 4′-chlorophenylimino-C-(methylthio)methyl-3-pyridine.
11. Use as claimed in claim 1 of 4′-chlorophenylimino-C-(tert.butylthio)methyl-3-pyridine.
12. Use as claimed in claim 1 of 4′-chlorophenylimino-C-(di-n-butylamino)methyl-3-pyridine.
13. Use as claimed in claim 1 of 4′-chlorophenylimino-C-(N-piperidinyl)methyl-3-pyridine.
14. A pesticidal and/or plant growth regulating composition comprising a surface active agent and optionally a carrier or a solid carrier and as pesticidal and/or plant growth regulating ingredient at least one compound of formula I in claim 1 as defined in any of claims 1 to 13, provided that when X is hydrogen, n is 0 or Z is methyl and n is 1, m = 1 and the derivative is a 2- or 4- pyridyl derivative then Y

is not methyl, methoxy, ethoxy or chloro; and when X is hydrogen and the derivative is a 6-methyl-2-pyridyl (n = 1) derivative or a 4-pyridyl (n = 0) derivative then m is not 0.

15. A process for the preparation of compounds of formula I in claim 1 other than those in which X is hydrogen or alkyl, as defined in claim 2, which a nicotinic anilide is reacted with a thionyl halide, and the formed phenylimino-C-(halo)methyl-3-pyridine is further converted by reaction with a compound QX, X having the aforesaid meaning with the exception of hydrogen and alkyl and Q being hydrogen, an alkali metal or copper.

16. A process as claimed in claim 15 wherein Q represents hydrogen or an alkali metal.

17. A process as claimed in claim 15 or 16 wherein the thionyl halide is thionyl chloride.

**Revendications pour les Etats contractants: BE CH DE FR GB IT LU NL**

1. L'utilisation comme fongicide et/ou régulateur de croissance des plantes d'un composé de formule:

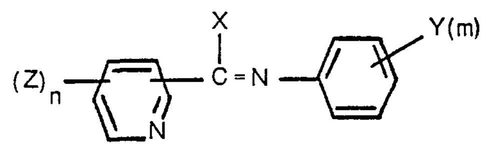

(I)

dans laquelle X représente un atome d'hydrogène, un groupe cyano, un groupe alcoxy non-substitué ou halogéné ou alcoxylé, un groupe cycloalkcoyloxy, un groupe alcoylthio, carboxyméthylthio, phénylthio, 2-chlorophénylthio, 4-chlorophénylthio, 4-bromophénylthio, 2,4-dichlorophénylthio, un groupe alcoyle ou un groupe —NRR', où R est un atome d'hydrogène ou un groupe alcoyle et R' est un groupe alcoyle, phényle ou cycloalcoyle et quand R est de l'hydrogène, R' peut être un groupe p-fluorophényle, benzyle, diisopropylaminoéthyle, méthoxyéthyle ou hydroxyéthyle ou R et R' en même temps que l'atome d'azote interjacent peuvent former un noyau hétérocyclique choisi parmi ceux de triazole, de pyrrolidine, de morpholine, de pipéridine ou de 3,5-diméthylpipéridine, Y et Z indépendamment sont des atomes d'hlogènes ou des groupes alcoyle, alcoxy ou nitro et m et n sont chacun 0, 1 ou 2, des groupes Y identiques ou différents pouvant être présents quand m = 2 et des groupes Z identiques ou différents quand n = 2.

2. L'utilisation selon la revendication 1 d'un composé de formule I qui est un dérivé 3-pyridyle.

3. L'utilisation selon la revendication 1 ou 2 d'un composé de formule I dans lequel X représente un groupe alcoxy.

4. L'utilisation selon la revendication 1 ou 2 d'un composé de formule I dans lequel X représente un groupe alcoylamino.

5. L'utilisation selon l'une quelconque des revendications 1 à 4 d'un composé de formule I dans lequel Y représente un atome d'halogène.

6. L'utilisation selon la revendication 1 de la 4'-chlorophénylimino-C-(cyano)méthyl-3-pyridine.

7. L'utilisation selon la revendication 1 de la 4'-chlorophénylimino-C-(n-propoxy)méthyl-3-pyridine.

8. L'utilisation selon la revendication 1 de la 4'-chlorophénylimino-C-(n-butoxy)méthyl-3-pyridine.

9. L'utilisation selon la revendication 1 de la 4'-chlorophénylimino-C-(cyclohexyloxy)méthyl-3-pyridine.

10. L'utilisation selon la revendication 1 de la 4'-chlorophénylimino-C-(méthylthio)méthyl-3-pyridine.

11. L'utilisation selon la revendication 1 de la 4'-chlorophénylimino-C-(tert-butylthio)méthyl-3-pyridine.

12. L'utilisation selon la revendication 1 de la 4'-chlorophénylimino-C-(di-n-butylamino)méthyl-3-pyridine.

13. L'utilisation selon la revendication 1 de la 4'-chlorophénylimino-C-(N-pipéridinyl)méthyl-3-pyridine.

14. Une composition pesticide et/ou régulatrice de croissance des plantes comprenant un agent tensio-actif et éventuellement un véhicule ou un véhicule solide et comme ingrédient pesticide et/ou régulateur de croissance des plantes au moins composé de la formule I de la revendication 1 comme défini dans l'une quelconque des revendications 1 à 13, avec les conditions que quand X est de l'hydrogène, n est 0 ou Z est un groupe méthyle et n est 1, m = 1 et le dérivé est un dérivé 2- ou 4-pyridyle, alors Y n'est pas un substituant méthyle, méthoxy, éthoxy ou chloro; et quand X est de l'hydrogène et le dérivé est un dérivé 6-méthyl-2-pyridyle (n = 1) ou un dérivé 4-pyridyle (n = 0), alors m n'est pas 0.

15. Des dérivés de phényliminométhylpyridine de la formule I de la revendication 1 comme définis dans la revendication 14, avec les conditions que quand X est del'hydrogène et n = 0, alors m n'est pas 0; et quand X est de l'hydogène et n = 0, alors m n'est pas 1 quand Y est un substituant chloro ou méthoxy; et quand X est de l'hydrogène, le dérivé est un dérivé 3- ou 4-pyridyle et m = 1, alors Y n'est

pas un substituant 3-nitro; et quand X est un groupe cyano, le dérivé est un dérivé 4-pyridyle, n = 0 et m = 1, alors Y est autre qu'un substituant 4-méthoxy.

16. Un procédé pour la préparation de composés de la formule I de la revendication 1 autres que ceux dans lesquels X est de l'hydrogène ou un groupe alcoyle, comme défini dans la revendication 2, dans lequel un anilide nicotinique est mis à réagir avec un halogénure de thionyle et la phénylimino-C-(halogéno)méthyl-3-pyridine formée est ensuite transformée par réaction avec un composé QX, X ayant la signification indiquée ci-dessus à l'exception de l'hydrogène et d'un groupe alcoyle et Q étant de l'hydrogène, un métal alcalin ou du cuivre.

17. Un procédé selon la revendication 16, dans lequel Q représente de l'hydrogène ou un métal alcalin.

18. Un procédé selon la revendication 16 ou 17, dans lequel l'halogénure de thionyle est le chlorure de thionyle.

**Revendications pour l'Etat contractant: AT**

1. L'utilisation comme fongicide et/ou régulateur de croissance des plantes d'un composé de formule:

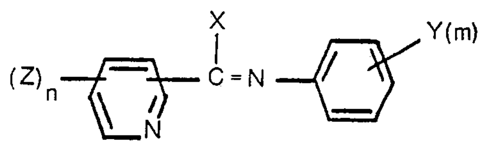

(I)

dans laquelle X représente un atome d'hydrogène, un groupe cyano, un groupe alcoxy non-substitué ou halogéné ou alcoxylé, un groupe cycloalkcoyloxy, un groupe alcoylthio, carboxyméthylthio, phénylthio, 2-chlorophénylthio, 4-chlorophénylthio, 4-bromophénylthio, 2,4-dichlorophénylthio, un groupe alcoyle ou un groupe —NRR', où R est un atome d'hydrogène ou un groupe alcoyle et R' est un groupe alcoyle, phényle ou cycloalcoyle et quand R est de l'hydrogène, R' peut être un groupe p-fluorophényle, benzyle, diisopropylaminoéthyle, méthoxyéthyle ou hydroxyéthyle ou R et R' en même temps que l'atome d'azote interjacent peuvent former un noyau hétérocyclique choisi parmi ceux de triazole, de pyrrolidine, de morpholine, de pipéridine ou de 3,5-diméthylpipéridine, Y et Z indépendamment sont des atomes d'halogènes ou des groupes alcoyle, alcoxy ou nitro, m et n sont chacun 0,1 ou 2, des groupes Y identiques ou différents pouvant être présents quand m = 2 et des groupes Z identiques ou différents quand n = 2.

2. L'utilisation selon la revendication 1 d'un composé de formule I qui est un dérivé 3-pyridyle.

3. L'utilisation selon la revendication 1 ou 2 d'un composé de formule I dans lequel X représente un groupe alcoxy.

4. L'utilisation selon la revendication 1 ou 2 d'un composé de formule I dans lequel X représente un groupe alcoylamino.

5. L'utilisation selon l'une quelconque des revendications 1 à 4 d'un composé de formule I dans lequel Y représente un atome d'halogène.

6. L'utilisation selon la revendication 1 de la 4'-chlorophénylimino-C-(cyano)méthyl-3-pyridine.

7. L'utilisation selon la revendication 1 de la 4'-chlorophénylimino-C-(n-propoxy)méthyl-3-pyridine.

8. L'utilisation selon la revendication 1 de la 4'-chlorophénylimino-C-(n-butoxy)méthyl-3-pyridine.

9. L'utilisation selon la revendication 1 de la 4'-chlorophénylimino-C-(cyclohexyloxy)méthyl-3-pyridine.

10. L'utilisation selon la revendication 1 de la 4'-chlorophénylimino-C-(méthylthio)méthyl-3-pyridine.

11. L'utilisation selon la revendication 1 de la 4'-chlorophénylimino-C-(tert-butylthio)méthyl-3-pyridine.

12. L'utilisation selon la revendication 1 de la 4'-chlorophénylimino-C-(di-n-butylamino)méthyl-3-pyridine.

13. L'utilisation selon la revendication 1 de la 4'-chlorophénylimino-C-(N-pipéridinyl)méthyl-3-pyridine.

14. Une composition pesticide et/ou régulatrice de croissance des plantes comprenant un agent tensio-actif et éventuellement un véhicule ou un véhicule solide et comme ingrédient pesticide et/ou régulateur de croissance des plantes au moins composé de la formule I de la revendication 1 comme défini dans l'une quelconque des revendications 1 à 13, avec les conditions que quand X est de l'hydrogène, n est 0 ou Z est un groupe méthyle et n est 1, m = 1 et le dérivé est un dérivé 2- ou 4-pyridyle, alors Y n'est pas un substituant méthyle, méthoxy, éthoxy ou chloro; et quand X est de l'hydrogène et le dérivé est un dérivé 6-méthyl-2-pyridyle (n = 1) ou un dérivé 4-pyridyle (n = 0), alors m n'est pas 0.

15. Un procédé pour la préparation de composés de la formula I de la revendication 1 autres que ceux dans lesquels X est de l'hydrogène ou un groupe alcoyle, comme défini dans la revendication 2,

selon lequel un anilide nicotinique est mis à réagir avec un halogénure de thionyle, et la phénylimino-C-(halogéno)méthyl-3-pyridine formée est transformée ensuite par réaction avec un composé QX, X ayant la signification indiquée ci-dessus à l'exception de l'hydrogène et d'un groupe alcoyle et Q étant de l'hydrogène, un métal alcalin ou du cuivre.

16. Un procédé selon la revendication 15, dans lequel Q représente de l'hydrogène ou un métal alcalin.

17. Un procédé selon la revendication 15 ou 16, dans lequel l'halogénure de thionyle est le chlorure de thionyle.

**Patentansprüche für die Vertragsstaaten: BE CH DE FR GB IT LU NL**

1. Verwendung einer Verbindung der Formel

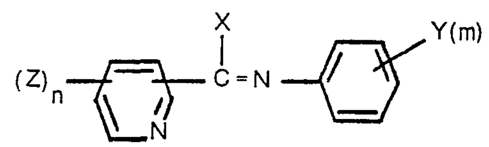

(I)

worin X für ein Wasserstoffatom, eine Cyanogruppe, eine unsubstituierte oder Halogen- oder Alkoxysubstituierte Alkoxygruppe eine Cycloalkyloxygruppe, eine Alkylthiogruppe, Carboxymethylthiogruppe, Phenylthiogruppe, 2-Chlorphenylthiogruppe, 4-Chlorphenylthiogruppe, 4-Bromphenylthiogruppe, 2,4-Dichlorphenylthiogruppe, eine Alkylgruppe oder eine Gruppe —NRR' steht, worin R ein Wasserstoffatom oder eine Alkylgruppe ist und R' eine Alkyl-, eine Phenyl- oder eine Cycloalkylgruppe bedeutet und, falls R für Wasserstoff steht, R' p-Fluorphenyl, Benzyl, Diisopropylaminoethyl, Methoxyethyl oder Hydroxyethyl sein kann oder R und R' gemeinsam mit dem dazwischen liegenden Stickstoffatom einen heterocyclischen Ring, ausgewählt unter Triazol, Pyrrolidin, Morpholin, Piperidin oder 3,5-Dimethylpiperidin, bilden können, Y und Z unabhängig voneinander für Halogenatome, Alkyl-, Alkoxy- oder Nitrogruppen stehen und m und n jeweils Null, 1 oder 2 bedeuten, wobei gleiche oder unterschiedliche Gruppen Y vorliegen können, falls m den Wert 2 hat, und gleiche oder unterschiedliche Gruppen Z vorliegen können, wenn n der Wert 2 hat, als ein Fungizid und/oder Pflanzenwachstumsregulator.

2. Verwendung einer Verbindung der Formel (I), welche eine 3-Pyridylderivat ist, wie in Anspruch 1 beansprucht.

3. Verwendung einer Verbindung der Formel (I), worin X eine Alkoxygruppe darstellt, wie in Anspruch 1 oder 2 beansprucht.

4. Verwendung einer Verbindung der Formel (I), worin X eine Alkylaminogruppe darstellt, wie in Anspruch 1 oder 2 beansprucht.

5. Verwendung einer Verbindung der Formel (I), worin Y ein Halogenatom darstellt, wie in einem der Ansprüche 1 bis 4 beansprucht.

6. Verwendung von 4'-Chlorphenylimino-C-(cyano)-methyl-3-pyridin wie in Anspruch 1 beansprucht.

7. Verwendung von 4'-Chlorphenylimino-C-(n-propoxy)-methyl-3-pyridin wie in Anspruch 1 beansprucht.

8. Verwendung von 4'-Chlorphenylimino-C-(n-butoxy)-methyl-3-pyridin wie in Anspruch 1 beansprucht.

9. Verwendung von 4'-Chlorphenylimino-C-(cyclohexyloxy)-methyl-3-pyridin wie in Anspruch 1 beansprucht.

10. Verwendung von 4'-Chlorphenylimino-C-(methylthio)-methyl-3-pyridin wie in Anspruch 1 beansprucht.

11. Verwendung von 4'-Chlorphenylimino-C-(tert.butylthio)-methyl-3-pyridin wie in Anspruch 1 beansprucht.

12. Verwendung von 4'-Chlorphenylimino-C-(di-n-butylamino)-methyl-3-pyridin wie in Anspruch 1 beansprucht.

13. Verwendung von 4'-Chlorphenylimino-C-(N-piperidinyl)-methyl-3-pyridin wie in Anspruch 1 beansprucht.

14. Eine pestizide und/oder pflanzenwuchsregulierende Zusammensetzung, umfassend ein oberflächenaktives Mittel und gegebenenfalls einen Träger oder einen festen Träger und als pestiziden und/oder pflanzenwuchsregulierenden Bestandteil wenigstens eine Verbindung der Formel (I) in Anspruch 1, wie in einem der Ansprüche 1 bis 13 definiert, mit der Maßgabe, daß, wenn X Wasserstoff ist, n den Wert Null hat oder Z Methyl ist und n den Wert 1 hat, m = 1 ist und das Derivat ein 2- oder 4-Pyridylderivat ist, dann Y nicht für Methyl, Methoxy, Ethoxy oder Chlor steht; und, falls X Wasserstoff bedeutet und das Derivat ein 6-Methyl-2-pyridyl (n = 1)-Derivat oder ein 4-Pyridyl (n = Null)-Derivat ist, dann m nicht für Null steht.

15. Phenyliminomethylpyridin-Derivate der Formel (I) in Anspruch 1, wie in Anspruch 14 definiert,

mit der Maßgabe, daß, falls X Wasserstoff ist und n den Wert Null hat, dann m nicht für Null steht; und, falls X Wasserstoff ist und n den Wert Null hat, dann m nicht den Wert 1 aufweist, wenn Y für Chlor oder Methoxy steht; und, falls X Wasserstoff ist, das Derivat ein 3- oder 4-Pyridyl-Derivat ist und m den Wert 1 hat, dann Y nicht ein 3-Nitrosubstituent ist; und, falls X eine Cyanogruppe ist, das Derivat ein 4-Pyridyl-Derivat ist und n den Wert Null und m den Wert 1 aufweisen, dann Y eine andere Bedeutung als einen 4-Methoxysubstituenten besitzt.

16. Ein Verfahren zur Herstellung von Verbindungen der Formel (I) in Anspruch 1, ausgenommen jene, in welchen X Wasserstoff oder Alkyl bedeutet, gemäß Definition in Anspruch 2, in welchem ein Nicotinsäureanilid mit einem Thionylhalogenid umgesetzt und das gebildete Phenylimino-C-(halogen)-methyl-3-pyridin weiter durch Umsetzung mit einer Verbindung QX umgewandelt wird, worin X die vorstehend angegebene Bedeutung, ausgenommen Wasserstoff und Alkyl, hat und Q für Wasserstoff, ein Alkalimetall oder Kupfer steht.

17. Ein Verfahren wie in Anspruch 16 beansprucht, worin Q Wasserstoff oder ein Alkalimetall bedeutet.

18. Ein Verfahren wie in Anspruch 16 oder 17 beansprucht, worin das Thionylhalogenid Thionylchlorid ist.

**Patentansprüche für den Vertragsstaat: AT**

1. Verwendung einer Verbindung der Formel

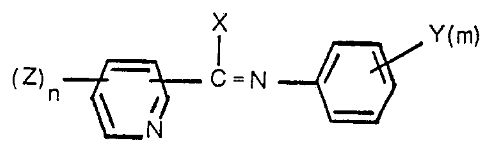

(I)

worin X für ein Wasserstoffatom, eine Cyanogruppe, eine unsubstituierte oder Halogen- oder Alkoxysubstituierte Alkoxygruppe, eine Cycloalkylgruppe, eine Alkylthiogruppe, Carboxymethylthiogruppe, Phenylthiogruppe, 2-Chlorphenylthiogruppe, 4-Chlorphenylthiogruppe, 4-Bromphenylthiogruppe, 2,4-Dichlorphenylthiogruppe, eine Alkylgruppe oder eine Gruppe —NRR′ steht, worin R ein Wasserstoffatom oder eine Alkylgruppe ist und R′ eine Alkyl-, eine Phenyl- oder eine Cycloalkylgruppe bedeutet und, falls R für Wasserstoff steht, R′ p-Fluorphenyl, Benzyl, Diisopropylaminoethyl, Methoxyethyl oder Hydroxyethyl sein kann oder R und R′ gemeinsam mit dem dazwischen liegenden Stickstoffatom einen heterocyclischen Ring, ausgewählt unter Triazol, Pyrrolidin, Morpholin, Piperidin oder 3,5-Dimethylpiperidin, bilden können, Y und Z unabhängig voneinander für Halogenatome, Alkyl-, Alkoxy- oder Nitrogruppen stehen und m und n jeweils Null, 1 oder 2 bedeuten, wobei gleiche oder unterschiedliche Gruppen Y vorliegen können, falls m den Wert 2 hat, und gleiche oder unterschiedliche Gruppen Z vorliegen können, wenn n der Wert 2 hat, als ein Fungizid und/oder Pflanzenwachstumsregulator.

2. Verwendung einer Verbindung der Formel (I), welche eine 3-Pyridylderivat ist, wie in Anspruch 1 beansprucht.

3. Verwendung einer Verbindung der Formel (I), worin X eine Alkoxygruppe darstellt, wie in Anspruch 1 oder 2 beansprucht.

4. Verwendung einer Verbindung der Formel (I), worin X eine Alkylaminogruppe darstellt, wie in Anspruch 1 oder 2 beansprucht.

5. Verwendung einer Verbindung der Formel (I), worin Y ein Halogenatom darstellt, wie in einem der Ansprüche 1 bis 4 beansprucht.

6. Verwendung von 4′-Chlorphenylimino-C-(cyano)-methyl-3-pyridin wie in Anspruch 1 beansprucht.

7. Verwendung von 4′-Chlorphenylimino-C-(n-propoxy)-methyl-3-pyridin wie in Anspruch 1 beansprucht.

8. Verwendung von 4′-Chlorphenylimino-C-(n-butoxy)-methyl-3-pyridin wie in Anspruch 1 beansprucht.

9. Verwendung von 4′-Chlorphenylimino-C-(cyclohexyloxy)-methyl-3-pyridin wie in Anspruch 1 beansprucht.

10. Verwendung von 4′-Chlorphenylimino-C-(methylthio)-methyl-3-pyridin wie in Anspruch 1 beansprucht.

11. Verwendung von 4′-Chlorphenylimino-C-(tert.butylthio)-methyl-3-pyridin wie in Anspruch 1 beansprucht.

12. Verwendung von 4′-Chlorphenylimino-C-(di-n-butylamino)-methyl-3-pyridin wie in Anspruch 1 beansprucht.

13. Verwendung von 4′-Chlorphenylimino-C-(N-piperidinyl)-methyl-3-pyridin wie in Anspruch 1 beansprucht.

14. Eine pestizide und/oder pflanzenwuchsregulierende Zusammensetzung, umfassend ein oberflächenaktives Mittel und gegebenenfalls einen Träger oder einen festen Träger und als pestiziden und/oder pflanzenwuchsregulierenden Bestandteil wenigstens eine Verbindung der Formel (I) in Anspruch 1, wie in einem der Ansprüche 1 bis 13 definiert, mit der Maßgabe, daß, wenn X Wasserstoff ist, n den Wert Null hat oder Z Methyl ist und n den Wert 1 hat, m = 1 ist und das Derivat ein 2- oder 4-Pyridylderivat ist, dann Y nicht für Methyl, Methoxy, Ethoxy oder Chlor steht; und, falls X Wasserstoff bedeutet und das Derivat ein 6-Methyl-2-pyridyl (n = 1)-Derivat oder ein 4-Pyridyl (n = Null)-Derivat ist, dann m nicht für Null steht.

15. Ein Verfahren zur Herstellung von Verbindungen der Formel (I) in Anspruch 1, ausgenommen jene, in welchen X Wasserstoff oder Alkyl bedeutet, gemäß Definition in Anspruch 2, in welchem ein Nicotinsäureanilid mit einem Thionylhalogenid umgesetzt und das gebildete Phenylimino-C-(halogen)-methyl-3-pyridin weiter durch Umsetzung mit einer Verbindung QX umgewandelt wird, worin X die vorstehend angegebene Bedeutung, ausgenommen Wasserstoff und Alkyl, hat und Q für Wasserstoff, ein Alkalimetall oder Kupfer steht.

16. Ein Verfahren wie in Anspruch 15 beansprucht, worin Q Wasserstoff oder ein Alkalimetall bedeutet.

17. Ein Verfahren wie in Anspruch 15 oder 16 beansprucht, worin das Thionylhalogenid Thionylchlorid ist.